(19) Europäisches Patentamt
European Patent Office
Office européen des brevets

(11) Veröffentlichungsnummer: **0 059 911**
**A1**

(12) # EUROPÄISCHE PATENTANMELDUNG

(21) Anmeldenummer: 82101532.8

(22) Anmeldetag: 27.02.82

(51) Int. Cl.³: C 07 H 23/00
A 61 K 31/70
//C07F15/00

(30) Priorität: 09.03.81 DE 3108842

(43) Veröffentlichungstag der Anmeldung: 15.09.82 Patentblatt 82/37

(84) Benannte Vertragsstaaten: AT CH DE FR GB IT LI SE

(71) Anmelder: BASF Aktiengesellschaft
Carl-Bosch-Strasse 38
D-6700 Ludwigshafen(DE)

(72) Erfinder: Beck, Wolfgang, Dr.
Melanchthon-Strasse 26
D-8000 München(DE)

(72) Erfinder: Thiel, Gerhard, Dr.
Elisabethstrasse 31
D-8000 München(DE)

(54) **Cis-Platin(II)-Komplexe mit Saccharid-Derivaten als Liganden.**

(57) Die Erfindung betrifft cis-Platin(II)-Komplexe, deren zwei Liganden Aminozucker oder mit Mono- oder Oligosacchariden verbundene Aminosäuren sind. Die Erfindung betrifft außerdem Heilmittel, die diese Komplexe als Wirkstoffe enthalten.

EP 0 059 911 A1

Croydon Printing Company Ltd.

# Cis-Platin(II)-Komplexe mit Saccharid-Derivaten als Liganden

Die Erfindung betrifft cis-Platin(II)-Komplexe, deren Liganden Saccharid-Derivate sind, nämlich Aminozucker oder mit Mono- oder Oligosacchariden verbundene Aminosäuren, Verfahren zu ihrer Herstellung und diese enthaltende Arzneimittel.

Es ist bekannt, daß cis-Platinkomplexe der allgemeinen Formel cis-$A_2PtX_2$ (wobei A ein neutraler Ligand, wie $NH_3$, organisches Amin, Peptid, X ein anionischer Ligand, wie Chlorid ist, Antitumorwirkung besitzen (DE-OS 26 26 559, 30 23 111). Cis-$(NH_3)_2PtCl_2$ ist als Arzneimittel eingeführt.

Es wurde nun gefunden, daß in cis-Platin(II)-Komplexen der Formel

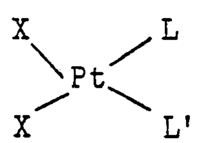 ,

in der L und L' über Aminogruppen an das Platin gebundene Liganden bedeuten, deren Aminogruppen Teile von Aminozuckern oder von mit Mono- oder Oligosacchariden verbundenen Aminosäuren sind, oder in der L und L' zusammengenommen den Rest einer mit Mono- oder Oligosacchariden verbundenen Thioaminosäure bedeuten und in der X für das Äquivalent eines therapeutisch vertretbaren Anions steht, die pharmakologischen und toxikologischen Eigenschaften der cytostatisch wirksamen Gruppe cis-$PtX_2$ günstig beeinflußt sind.

B/P

Eine Erklärung für diesen Effekt könnte darin gesehen werden, daß diese neutralen Liganden, die Bausteine der in den Zellwänden auftretenden Glycoproteine sind, als Carrier für den Transport der cytostatisch wirksamen cis-$PtX_2$-Gruppe zu einer Anreicherung von Platin in der Tumorzelle führen. Denn bei neoplastischen Geweben erfolgt ein verstärkter Transport von höhermolekularen Verbindungen durch die Membran.

Die als Liganden L bzw. L' vorhandenen Aminozucker können sich von Mono- wie von Oligosacchariden herleiten. Es können Pyranosen und Furanosen sein. Bevorzugt sind Mono- und Disaccharide, insbesondere Hexosen, doch kommen Pentosen ebenfalls in Betracht. Die Aminozucker können auch mehr als eine freie Aminogruppe haben. Ihre Hydroxylgruppen können durch Veresterung, Veretherung oder Ketalisierung geschützt sein, wodurch sich Löslichkeitsverhalten, die chemischen und biochemischen Eigenschaften der Platinkomplexe über einen weiten Bereich variieren lassen. Beispiele für Aminozucker sind: 2-Amino-2-deoxy-D-galactopyranose, 2-Amino-2-deoxy-D-mannopyranose, 2-Amino-2-deoxy-D-altrose, 2-Amino-2-deoxy-α-D-ribose, 2-Amino-2-deoxy-α-D-allose, 2-Amino-2-deoxy-D-gulose, 2-Amino-2-deoxy-α-D-talose, Chitobiose (N,N'-Diacetylchitobiose), 4-Amino-1,2-dihydro-1ß-D-ribofuranosyl-1,3,5-triazin-2-on, 2-Deoxy-2-amino-palatinose (2-Deoxy-2-amino-6-O-α-D-glucopyranosyl-D-fructose), 2,3-Diamino-2,3-dideoxy-α-D-glucose, 2,6-Diamino-2,6-dideoxy-α-D-galactose, 2,6-Diamino-2,6-dideoxy-ß-D-mannose und ihre durch Veresterung, Veretherung oder Ketalisierung erhaltenen Derivate.

Aufgrund der leichten Zugänglichkeit und ihrer Bedeutung in der Biosynthese der Zelle bieten sich insbesondere Derivate der 2-Amino-2-deoxy-D-glucose (D-Glucosamin) und der D-Galactopyranose an, z.B.

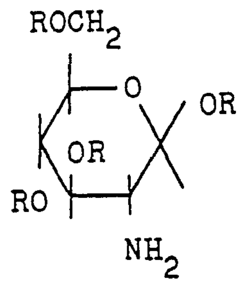

R=$CH_3CO$-

1

2-Amino-2-deoxy-1,3,4,6-tetra-O-acetyl-ß-D-glucopyranose

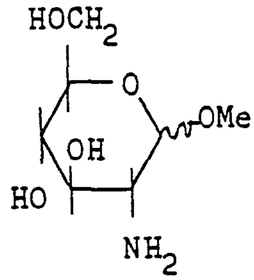

3

O-Methyl-2-amino-2-deoxy-α,ß-D-glucopyranosid

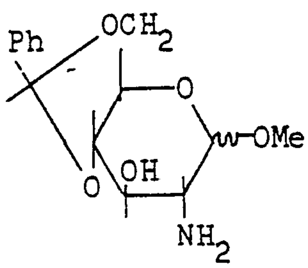

2

O-Methyl-2-amino-deoxy-4,6-O-benzyliden-α,ß-D-glucopyranosid

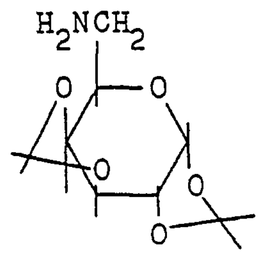

4

6-Amino-6-deoxy-1,2:3,4-di-O-isopropyliden-α-D-galactopyranose

Therapeutisch vertretbare Anionen X sind insbesondere $Cl^-$, $NO_3^-$, $SO_4^{2-}$, Malonat, Hydroxymalonat, Ethylmalonat, Oxalat, Gluconat, 1,1-Cyclobutandicarboxylat, Phthalat, 4-Carboxyphthalat, Benzolsulfonat.

Die erfindungsgemäßen Platinkomplexe können z.B. erhalten werden durch Umsetzung von $K_2PtCl_4$ mit dem betreffenden Aminozucker in wäßriger oder Wasser/Tetrahydrofuran (THF)-Lösung, wobei die Mengen der Umsetzungspartner derart gewählt werden, daß sich das Umsetzungsverhältnis 1 Mol Pt-Ausgangskomplex : 2 Mol Aminozucker ergibt. Nach Abziehen des THF und Extraktion mit $CH_2Cl_2$ liegen die Komplexe als nahezu farblose Pulver vor.

$$K_2PtCl_4 + 2\ L \xrightarrow[-2\ KCl]{20\ h,\ RT} L_2PtCl_2$$

Andere Flüssigkeiten, in denen die Umsetzung vorgenommen werden kann, sind Methanol, Dimethylsulfoxid, Dimethylformamid, Dioxan, Ethylenglykoldimethylether, Diethylenglykoldimethylether und ihre Gemische oder Mischungen aus diesen Lösungsmitteln mit Wasser. Zur Extraktion der Verbindungen kann anstelle von $CH_2Cl_2$ auch ein anderes mit Wasser nicht mischbares, polares Lösungsmittel wie z.B. $CHCl_3$, Diethylether, verwendet werden.

Die Umsetzungstemperatur kann im bevorzugten Bereich von ungefähr 20°C bis ungefähr 50°C liegen. Die Dauer der Umsetzung beträgt je nach Temperatur und Reaktionspartner z.B. 12 bis 24 Stunden.

Da Monosaccharide mit freier hemiacetalischer OH-Gruppe reduzierend wirken, empfiehlt es sich, zur Darstellung von Platinkomplexen Derivate einzusetzen, bei denen die Funktion blockiert ist, z.B. Glycoside oder Ester. Andernfalls könnte Platin(II) zum Platinmetall reduziert werden.

Üblicherweise entstehen bei Raumtemperatur die cis-Isomeren, jedoch können sterisch anspruchsvolle Aminozucker bereits unter diesen schonenden Bedingungen zu einem Gemisch aus cis- und trans-Form führen.

Um auch sterisch anspruchsvolle Aminozucker ausschließlich in die cis-Position zu dirigieren, wird besser statt $K_2PtCl_4$ das entsprechende Tetraiodoplatinat $K_2PtJ_4$ (in situ dargestellt aus $K_2PtCl_4$ und einem Überschuß an KJ in wäßriger Lösung) verwendet. Jodid ist eine besser austretende Gruppe und übt einen stärkeren trans-Effekt als Chlorid aus.

$$K_2PtCl_4 + 4\ KJ \xrightarrow[-\ 4\ KCl]{} K_2PtJ_4 \xrightarrow[-2\ KJ]{+2\ L;\ 20\ h,\ RT} cis\text{-}L_2PtJ_2$$

Die cytostatische Aktivität der Jodo-Platin(II)-Komplexe ist für manche Zwecke zu gering. Daher empfiehlt es sich, das Jodid mittels der Silberhalogenidabstraktion gegen andere anionische Liganden auszutauschen. Dabei bleibt die cis-Konfiguration erhalten. Der Austausch kann mit wäßriger $AgNO_3$-Lösung erfolgen, was zum ionischen Diaquokomplex $[L_2Pt(H_2O)_2](NO_3)_2$ führt. Der schwache Ligand Wasser kann leicht mit stärkeren Donoren (z.B. $Cl^-$, $Br^-$, $Malonat^{2-}$ u.ä.) verdrängt werden.

$$L_2PtJ_2 + 2\ AgNO_3 \xrightarrow[-2\ AgJ]{20\ h,\ RT;\ H_2O} [L_2Pt(H_2O)_2](NO_3)_2$$

$$\downarrow + Cl^{\ominus}$$

$$L_2PtCl_2$$

Der Austausch kann auch unmittelbar in wasserfreiem Medium mit Silbersalzen geeigneter Liganden, wie $Ag_2SO_4$ oder $AgNO_3$ erfolgen. Auf diese Weise werden z.B. die beiden schwachgelben Nitrato- und Sulfato-Verbindungen gewonnen:

$$L_2PtJ_2 + AgX \xrightarrow[-2\ AgJ]{2d,\ RT;\ CH_2Cl_2} L_2PtX_{(2)}$$

$$L = \underline{\underline{1}} : X = NO_3^{\ominus}\ ;\quad L = \underline{\underline{2}} : X = SO_4^{2\ominus}$$

Die Synthese der Komplexe aus $K_2PtCl_4$ bzw. $K_2PtJ_4$ im Wasser/THF-Gemisch macht infolge der beträchtlichen Wasserlöslichkeit dieser Komplexe zur Steigerung der Ausbeute über 60 % d.Th. häufig eine zusätzliche Aufarbeitung der wäßrigen

Reaktionslösung wünschenswert. So läßt sich durch schonendes Eindunsten im Exsikkator über $H_2SO_4$ und anschließende Extraktion mit $CH_2Cl_2$ die Ausbeute steigern, was wegen der aufwendigen Darstellung der verwendeten Aminozucker von Bedeutung sein kann. Der Komplex $L_2PtCl_2$ des wasserlöslichen Aminozuckers 3 (s. die Formeln auf Seite 3) wird durch Reaktion mit $K_2PtCl_4$ in Wasser und langsames Ausfällen mit Aceton als sehr gut wasserlösliche, farblose Verbindung gewonnen.

Die Pt-Komplexe mit Aminosäuren können im Falle der α-Aminosäuren auch wie folgt wiedergegeben werden:

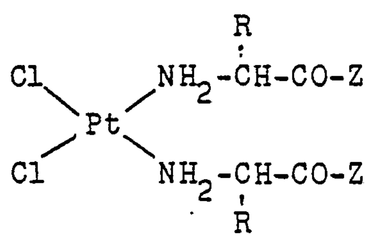

worin Z ein über eine Ester- oder eine Amidgruppe gebundenes Mono- oder Oligosaccharid bedeutet und R für Wasserstoff oder einen in Aminosäuren üblichen Rest steht.

Als Aminosäuren, die Teile der Liganden L und L' sein können, sind vorzugsweise die L-Formen der natürlich vorkommenden α-Aminosäuren zu nennen, insbesondere Glycin, Alanin, Valin, Leucin, Isoleucin, Phenylalanin, Tyrosin, Threonin, Serin, Methionin, Prolin, Histidin, S-Alkylcystein. Jedoch kommen auch die Racemate der α-Aminosäuren sowie andere Aminosäuren, z.B. ß-Aminosäuren, wie ß-Alanin in Betracht.

Aminosäure und Mono- oder Oligosaccharide können durch eine Ester- oder eine Amidgruppe verbunden sein, d.h., das Saccharid stellt die Alkohol- bzw. Aminkomponente dar. Im letzten Fall handelt es sich demnach um einen Aminozucker.

Für die Saccharidkomponenten, die mit der Carboxylgruppe der Aminosäuren verbunden sind, gelten die selben Angaben bezüglich Vielfalt wie für die Aminozucker als Liganden L und L'. Zur Herstellung dieser Platin(II)-Komplexe mit Aminosäure-sacchariden verknüpft man N-koordinierte Aminosäuren mit einer freien OH-Gruppe von Zucker oder der $NH_2$-Gruppe von Aminozuckern unter Verwendung eines in der Peptidchemie üblichen Kupplungsreagenz, vorzugsweise Carbodiimid.

Ausgehend von den leicht zugänglichen Aminosäurekomplexen mit freien Carboxylgruppe cis-$(GlyOH)_2PtCl_2$ sowie $(MethOH)PtCl_2$ und den Zuckern 1,3,4,6-Tetra-O-acetyl-ß-D-glucosamin, O-Methyl-N-acetyl-2-amino-2-deoxy-α,ß-D-glucopyranosid und 1,2:3,4-Di-O-isopropyliden-α-D-galactopyranose werden Platin(II)-Komplexe mit O- oder N-verknüpften Glycosylaminosäuren als Liganden erhalten:

$$Cl_2Pt(NH_2CHRCO_2H)_2 + R'NH_2 \xrightarrow[-DCH]{+ DCCD,\ DMF/RT} Cl_2Pt(NH_2CHRCONHR')_2$$

$$Cl_2Pt(NH_2CHRCO_2H)_2 + R'OH \xrightarrow[-DCH]{+ DCCD,\ DMF/RT} Cl_2Pt(NH_2CHRCO_2R')_2$$

Beispiele solcher Komplexe sind:

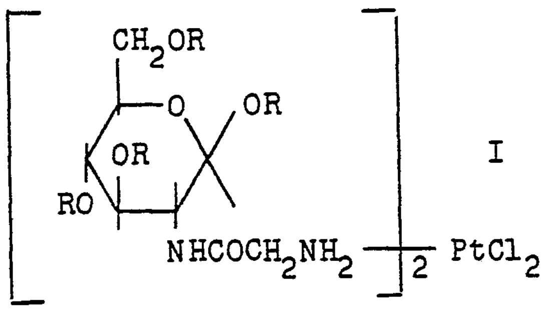

I

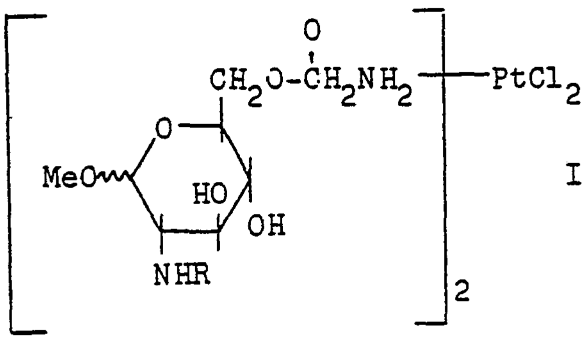

II

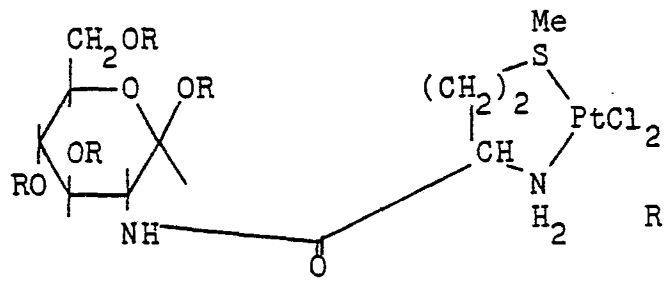

III

R = $CH_3CO-$

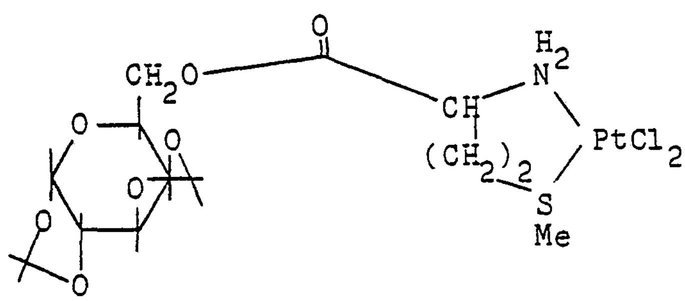

IV

Außer dem als Lösungsmittel bevorzugten Dimethylformamid (DMF) kommen beispielsweise in Betracht Dimethylsulfoxid, Tetrahydrofuan, Dioxan, Ethylenglykoldimethylether, Diethylenglykoldimethylether, Methanol und ihre Gemische.

Es ist auch möglich, die Aminosäure zunächst mit Zucker zu verestern bzw. eine Aminosäure, deren Aminogruppe in üblicher Weise zunächst geschützt ist, mit Aminozucker in das Amid überzuführen und dann den Platinkomplex in der bei Aminozuckern angegebenen Weise zu bilden. Im übrigen können die in der DE-OS 26 26 559 beschriebenen Maßnahmen sinngemäß auch hier angewendet werden.

Durch Variation der OH-Schutzgruppen läßt sich auch hier die Löslichkeit dieser Komplexe in verschiedenen Lösungsmitteln steuern.

Wie die eingangs wiedergegebene Formel mit den Liganden L und L' zeigt, müssen diese nicht identisch sein, auch Mischkomplexe gehören zum Gegenstand dieser Erfindung und können hergestellt werden. L und L' können zusammengenommen auch für eine Aminosäure stehen, die eine S-Alkyl-Gruppe enthält, sofern dieser Aminosäurerest ebenfalls mit einem Mono- oder Oligosaccharid über ein Ester- oder Amidglied verbunden ist.

Beispiel 1

Darstellung der Platin(II)-Aminozuckerkomplexe mit den Liganden 2 und 4

0,41 g (1 mmol) $K_2PtCl_4$ werden in möglichst wenig Wasser (2 bis 3 ml) gelöst. Zu dieser Lösung wird eine konzentrierte Lösung des entsprechenden Aminozuckers (2 mmol) in THF (2-3 ml) gegeben. Der ausgefallene Niederschlag wird nun durch Zugabe von Wasser gerade wieder aufgelöst. Die hellrote Reaktionslösung wird 20 Stunden bei Raumtemperatur (RT) gerührt. Der ausgefallene, teilweise ölige Niederschlag wird abgetrennt und aus $CH_2Cl_2/Et_2O$/Pentan umgefällt. Das in der Mutterlauge enthaltene THF wird im Vakuum abgezogen. Die dabei ausgefallenen gelben Öle

werden mit $CH_2Cl_2$ aus der wäßrigen Reaktionsphase extrahiert (3 x 10 ml). Trocknen mit $Na_2SO_4$, Abdestillieren des $CH_2Cl_2$ unter vermindertem Druck und Umfällen aus $CH_2Cl_2$/$Et_2O$/Pentan liefert weiteres Reaktionsprodukt.
Ausbeuten: 75 bis 80 % d.Th.
Löslichkeit: sehr gut löslich in THF, $CH_2Cl_2$, Alkoholen, etwas löslich in $Et_2O$.

Beispiel 2

Platinkomplex cis-$L_2PtCl_2$ mit dem Liganden 1

0,41 g (1 mmol) $K_2PtCl_4$ werden in möglichst wenig Wasser (2 ml) gelöst. Zu dieser Lösung werden 2 mmol des Aminozuckers 1 in 2 ml THF gegeben. Der ausgefallene Niederschlag wird durch Zugabe von Wasser vorsichtig gerade wieder aufgelöst und die Reaktionslösung 20 Stunden bei RT gerührt. Nach Abziehen des THF im Vakuum wird die wäßrige Phase mit $CH_2Cl_2$ extrahiert (4 x 15 ml). Nach Trocknen mit $Na_2SO_4$ und Abdestillieren des Lösungsmittels unter vermindertem Druck wird das gelbe Öl mit $Et_2O$/ Pentan verrührt. Vom entstandenen Feststoff wird abgesaugt, und aus $CH_2Cl_2$/$Et_2O$/Pentan umgefällt. Die Ausbeute an farblosem Komplex beträgt 30 bis 40 % d.Th., da dieser merklich wasserlöslich ist. Durch Eindunsten der wäßrigen Phase im Exsikkator über konz. $H_2SO_4$ und Extraktion des festen Rückstandes mit $CH_2Cl_2$ lassen sich weitere Mengen Substanz erhalten (ca. 20 % d.Th.).
Löslichkeit: Die Verbindung zeigt eine Verteilung zwischen $CHCl_3$/$H_2O$ oder n-Octanol/$H_2O$ von ca. 60 : 40.

IR: 3230, 3135, 1750, 1740, 1578, 1367, 1220, 1055, 1045, 395, 345, 335, 330, 288 $cm^{-1}$ (in Hostaflon - Warenzeichen der Firma Hoechst Aktiengesellschaft - bzw. KBr)

$^1H$-NMR: 6,32d, 6, 16q, 5,03q, 4.6-3,8 m, 2,29 s, 2,06s. ($\delta$ in ppm mit ThS als internem Standard, Lösung in $CDCl_3$).

$^{13}C$-NMR: 170.6, 170.1, 160.6, 168.6; 91.7, 73.1, 71.9, 68.6, 61.7, 58.2; 21.5, 21.3, 20.7 (in $CDCl_3$, $_c$ in ppm bezogen auf TMS).

Die Verteilung des Dichlorokomplexes mit dem Aminozucker 1 zwischen Chloroform/Wasser oder n-Octanol/Wasser beträgt ungefähr 60 : 40. Dies ist im Hinblick auf die physiologische Aktivität (Verteilung zwischen Lipid- und wäßriger Phase) von Interesse.

Beispiel 3

Darstellung der Jodo-Komplexe $L_2PtJ_2$ mit den Liganden 1 und 2

0,41 g (1 mmol) $K_2PtCl_4$ und 1,66 g (10 mmol) Kaliumjodid ergeben in 6 ml Wasser nach 30 Minuten bei RT eine tiefbraune Lösung von $K_2PtJ_4$. Zu dieser wird eine konzentrierte Lösung des entsprechenden Aminozuckers in THF (3—4 ml) gegeben und dann gerade soviel Wasser bis sich der entstandene Niederschlag wieder aufgelöst hat. Das Reaktionsgemisch wird 20 Stunden bei RT unter Lichtausschluß gerührt. Nach Abziehen des THF im Vakuum wird das ölige, abgeschiedene Reaktionsprodukt mit $CH_2Cl_2$ extrahiert (3x20 ml), die $CH_2Cl_2$-Phase mit $Na_2SO_4$ getrocknet, im Vakuum eingeengt und mit $Et_2O$/Pentan gefällt. Nach Umfällen aus $CH_2Cl_2$/$Et_2O$/Pentan werden dunkelgelbe Pulver erhalten.

Ausbeuten: mit L=1: 40 % d.Th.
mit L=2: 80 bis 90 % d.Th.

Löslichkeit: analog den entsprechenden Chloro-Kompolexen; die Ausbeute mit L=1 ist wegen der beträchtlichen Wasserlöslichkeit der Verbindung vermindert und kann durch Aufarbeitung, wie im Beispiel 2 beschrieben, etwas verbessert werden. Bei diesen Verbindungen ist es empfehlenswert, während der Aufarbeitung Temperaturen über 50°C zu vermeiden, da es sonst zu Zersetzungsreaktionen unter Metall- und Jodabscheidung kommen kann.

Beispiel 4

Cis-$L_2PtCl_2$ mit L=1

1 mmol des entsprechenden Jodokomplexes mit L=1 werden in 5 ml THF gelöst und mit exakt 2 mmol $AgNO_3$ in 1 ml Wasser versetzt. Das Reaktionsgemisch wird unter Lichtausschluß 20 Stunden bei RT gerührt. Danach wird das gelbe Silberjodid abfiltriert, mit wenig Wasser gewaschen und die so erhaltene gelbe Reaktionslösung mit einem Überschuß an KCl versetzt. Nach Rühren bei RT über Nacht wird die Lösung wie bei Beispiel 2 aufgearbeitet. Die erhaltene farblose Verbindung ist mit der in Beispiel 2 beschriebenen identisch (Ausbeute: 30 bis 40 % d.Th.).

Beispiel 5

cis-$L_2Pt(NO_3)_2$ und cis-$L_2Pt(SO_4)$ (L=1, 2)

1 mmol des entsprechenden Jodokomplexes mit L=1 oder 2 werden in 5 bis 10 ml abs. $CH_2Cl_2$ gelöst und mit genau 2 mmol $AgNO_3$ oder 1 mmol $Ag_2SO_4$ versetzt. Die Suspensionen werden mehrere Tage bei RT unter Lichtausschluß gerührt. Danach wird das ausgefallene gelbe AgJ abfiltriert, mit $CH_2Cl_2$ gewaschen und von der erhaltenen gelben Reaktionslösung das Lösungsmittel weitgehend im Vakuum abgezogen. Durch Fällen mit $Et_2O$/Pentan und nach mehrmaligem Umfällen aus $CH_2Cl_2$/$Et_2O$/Pentan werden so die hellgelben Komplexe gewonnen (Tocknung im Vakuum über konz. $H_2SO_4$).
Ausbeuten: 60 bis 70 % d.Th.

Das Löslichkeitsverhalten ist ähnlich dem anderer Aminozuckerkomplexe.

Beispiel 6

cis-$L_2PtCl_2$ mit L=3

Zu 1 mmol $K_2PtCl_4$ in 4 ml $H_2O$ werden 2 mmol des Zuckers 3 in 1 ml $H_2O$ gegeben. Nach 20 Stunden Rühren bei RT wird aus der nun hellgelben Reaktionslösung durch Zugabe von 30 ml Aceton und Kühlen auf -15°C die farblose Verbindung langsam ausgefällt. Nach einigen Stunden bei -15°C werden die Niederschläge abgefrittet und sofort mit viel Aceton gewaschen (Trocknung über $P_2O_5$ im Vakuum).
Ausbeuten: 40 bis 50 % d.Th.
Löslichkeit: sehr gut löslich in $H_2O$, EtOH, MeOH
IR: 3400, 3240, 1570, 1365, 1210, 1125, 1085, 1035, 325 $cm^{-1}$ (in Nujol).

Darstellung der Platinkomplexe, in denen die Saccharidkomponenten über α-Aminosäuren gebunden sind

Beispiel 7

Darstellung von Komplex I

1 mmol cis-$(GlyOH)_2PtCl_2$ werden in 3 ml abs. DMF gelöst. Die hellgelbe Lösung wird zuerst mit 2 mmol Dicyclohexylcarbodiimid und dann mit 2 mmol des Aminozuckers 1 versetzt. Nach einer Minute beginnt die Reaktion unter Abscheidung von farblosem und fein verteiltem Dicyclohexylharnstoff sowie leichter Erwärmung. Nach 5 bis 6 Stunden bei RT wird der abgeschiedene Harnstoff abgesaugt. Entfernen des DMF im Vakuum ergibt ein gelbes Öl, das mit 2 bis 3 ml THF aufgenommen wird. Der restliche Harnstoff wird nun durch Filtration über eine kurze Celluloseschicht abgetrennt. Abziehen des THF im Vakuum und Verrühren des öligen Rück-

standes mit $Et_2O$/Pentan liefert farblose Substanz. Diese wird aus EtOH/$Et_2O$ umgefällt (Trocknung im Vakuum über konz. $H_2SO_4$).
Ausbeuten: 60 % d.Th.
Löslichkeit: gut löslich in den üblichen polaren organischen Lösungsmitteln; etwas wasserlöslich.
IR: 3340, 3270, 3230, 1750, 1688, 1580, 1545, 1376, 326 $cm^{-1}$ (in KBr).

Beispiel 8
Darstellung von Komplex II

Die Umsetzung wird wie bei Beispiel 7 durchgeführt mit 6 ml abs. DMF und Methyl-N-acetyl-2-amino-2-doxy-α,ß-D-glucopyranosid als Zuckerkomponente. Nach 20 Stunden Rühren bei RT wird der abgeschiedene Harnstoff abfiltriert, das Lösungsmittel im Vakuum entfernt und das erhaltene gelbe Öl in 4 ml EtOH aufgenommen. Nach 6 Stunden bei -15°C wird der restliche Harnstoff durch Filtration über eine kurze Celluloseschicht abgetrennt und aus der Reaktionslösung vorsichtig mit $Et_2O$ und Kühlen auf -15°C nahezu farbloses Pulver gefällt. Die Reaktionsprodukte werden abgefrittet und mit $Et_2O$ gewaschen (Trocknung im Vakuum über konz. $H_2SO_4$).
Ausbeuten: 60 bis 70 % d.Th.
Löslichkeit: gut löslich in $H_2O$, MeOH, EtOH; etwas löslich in THF
IR: 3350, 3285, 3220, 3130, 1737, 1650, 1582, 1554, 1376, 324 $cm^{-1}$ (in KBr).

Beispiel 9
Darstellung der Komplexe III und IV

Zu 1 mmol (MethOH)$PtCl_2$ in 3 ml abs. DMF werden 1 mmol Dicyclohexylcarbodiimid und anschließend 1 mmol Aminozucker 1 bzw. 1,2:3,4-Di-O-isopropyliden-α-D-galactopyranose gegeben.

Nach 30 Sekunden beginnt die Reaktion unter Abscheidung von Dicyclohexylharnstoff und schwacher Erwärmung des Reaktionsgemisches. Der abgeschiedene Harnstoff wird nach 20 Stunden bei RT abgesaugt, das DMF im Vakuum abgezogen und das erhaltene gelbe Öl in 3 ml THF aufgenommen. Nach mehrstündigem Kühlen auf -15°C wird der restliche Harnstoff abfiltriert, das THF im Vakuum abgezogen und der Rückstand mit $Et_2O$/Pentan verrührt. Die so erhaltenen hellgelben pulvrigen Produkte werden abgefrittet, mit $Et_2O$/Pentan gewaschen und im Vakuum über konz. $H_2SO_4$ getrocknet.
Ausbeuten: 70 bis 80 % d.Th.
Löslichkeit: sehr gut löslich in üblichen polaren organischen Lösungsmitteln
IR von III: 3320, 3245, 3110, 1750, 1685, 1570, 1540, 1365, 328, 312 $cm^{-1}$
IR von IV: 3250, 3190, 3115, 1744, 1573, 1382, 1375, 330 312 $cm^{-1}$ (in KBr bzw. Nujol).

Die erfindungsgemäßen Platinkomplexe zeichnen sich aufgrund ihrer cytostatischen Wirkung durch wertvolle pharmakologische Eigenschaften aus. Sie können daher als Chemotherapeutika bei der Behandlung von Krebs Verwendung finden. Dementsprechend sind ein weiterer Gegenstand der vorliegenden Erfindung therapeutische Mittel, enthaltend neben üblichen Träger und Verdünnungsmitteln mindestens einen der erfindungsgemäßen Platinkomplexe als Wirkstoff. Die Mittel können je nach Zubereitung intravenös, intramuskulär, intraperitoneal, subkutan oder peroral verabreicht werden. Auch äußerliche Applikation ist möglich. Die Mittel können in Form von Lösungen oder Suspensionen in Waser oder einem polaren organischen Lösungsmittel vorliegen, wenn nicht andere, ebenfalls mögliche Zubereitungsformen gewünscht werden.

Tabelle 1 Analytische Daten der Aminozuckerkomplexe

| Verbindung | Summenformel (Molmasse) | ber. gef. | C | H | N | Farbe | Zersetzung ab (°C) |
|---|---|---|---|---|---|---|---|
| $L_2PtCl_2$; L=1 | $PtC_{28}H_{42}Cl_2N_2O_{18}$ (960,6) | ber. | 35,01 | 4,41 | 2,92 | farblos | 115 |
| | | gef. | 35,62 | 4,97 | 2,91 | | |
| $L_2PtCl_2$; L=2 | $PtC_{28}H_{38}Cl_2N_2O_{10}$ (828,5) | ber. | 40,59 | 4,62 | 3,38 | farblos | 168 |
| | | gef. | 40,43 | 4,86 | 3,26 | | |
| $L_2PtCl_2$; L=4 | $PtC_{24}H_{42}Cl_2N_2O_{10}$ (784,6) | ber. | 36,74 | 5,40 | 3,57 | farblos | 137 |
| | | gef. | 37,96 | 5,67 | 3,63 | | |
| $L_2PtCl_2$; L=3 | $PtC_{14}H_{30}Cl_2N_2O_{10}$ (652,3) | ber. | 25,78 | 4,64 | 4,29 | farblos | 139 |
| | | gef. | 25,58 | 4,50 | 3,89 | | |
| $L_2PtJ_2$; L=1 | $PtC_{28}H_{42}J_2N_2O_{18}$ (1143,5) | ber. | 29,41 | 3,70 | 2,45 | gelb | 122 |
| | | gef. | 28,06 | 4,08 | 2,36 | | |
| $L_2PtJ_2$; L=2 | $PtC_{28}H_{38}J_2N_2O_{10}$ (1011,4) | ber. | 33,25 | 3,79 | 2,77 | gelb | 175 |
| | | gef. | 32,77 | 3,63 | 2,66 | | |
| $L_2PtSO_4$; L=2 | $PtC_{28}H_{38}N_2O_{14}S$ (854,2) | ber. | 39,37 | 4,48 | 3,28 | hellgelb | 147 |
| | | gef. | 38,43 | 4,84 | 3,16 | | |
| $L_2Pt(NO_3)_2$; L=1 | $PtC_{28}H_{42}N_4O_{24}$ (1013,5) | ber. | 33,18 | 4,18 | 5,53 | hellgelb | 123 |
| | | gef. | 33,29 | 4,40 | 5,23 | | |

Tabelle 2 Analytische Daten der Glycosylaminosäurekomplexe

| Verbindung | Summenformel (Molmasse) | ber. gef. | C | H | N | Farbe | Zersetzung ab ($^{o}C$) |
|---|---|---|---|---|---|---|---|
| I | $PtC_{32}H_{48}Cl_2N_4O_{20}$ (1074,7) | ber. | 35,76 | 4,50 | 5,21 | farblos | 170 |
| | | gef. | 35,19 | 4,88 | 5,15 | | |
| II | $PtC_{22}H_{40}Cl_2N_4O_{14}$ (850,6) | ber. | 31,07 | 4,74 | 6,59 | farblos | 155 |
| | | gef. | 32,70 | 5,73 | 6,64 | | |
| III | $PtC_{19}H_{30}Cl_2N_2O_{10}S$ (744,5) | ber. | 30,69 | 4,06 | 3,76 | hellgelb | 129 |
| | | gef. | 31,06 | 4,35 | 4,02 | | |
| IV | $PtC_{17}H_{29}Cl_2NO_7S$ (657,5) | ber. | 31,05 | 4,45 | 2,13 | hellgelb | 115 |
| | | gef. | 29,87 | 4,65 | 2,67 | | |

Nach einem Verfahren, das in der Zeitschrift Archiv der Pharmazie 314. Band, Heft 11, Seiten 955-967, dort eingegangen 5. Februar 1981, beschrieben ist, wurden Verbindungen dieser Erfindung auf Hemmung des 3H-Thymidin--Einbaus in die Zelle getestet. Das Maß der Hemmung entspricht einer Hemmung des Zellwachstums. Ein Ergebnis wird hier wiedergegeben:

In vitro-Testergebnisse an der hormonunabhängigen menschlichen MDA-MB 231 Mammatumorzellkultur

| Verbindung | Konzentration | Hemmung des 3H-Thymidin-Einbaus |
|---|---|---|
| 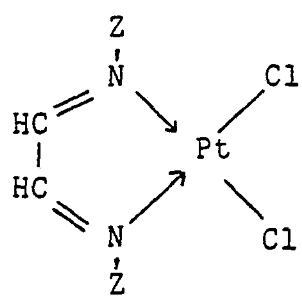 | $10^{-5}$ m | 65,8 % |
| | $10^{-6}$ m | 12,1 % |

Z = 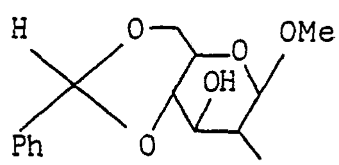

Patentansprüche

1. Cis-Platin(II)-Komplexe der Formel

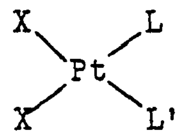

in der L und L' über Aminogruppen an das Platin gebundene Liganden bedeuten, deren Aminogruppen Teile von Aminozuckern oder von mit Mono- oder Oligosacchariden verbundenen Aminosäuren sind, oder in L und L' zusammengenommen den Rest einer mit Mono- oder Oligosacchariden verbundenen Thioaminosäure ist, und in X für das Äquivalent eines therapeutisch vertretbaren Anions steht.

2. Cis-Platin(II)-Komplexe gemäß Anspruch 1, in denen reduzierend wirkende OH-Gruppen der Aminozucker bzw. der mit Aminosäuren verbundenen Mono- oder Oligosaccharide durch Veresterung, Veretherung oder Ketalisierung blockiert sind.

3. Cis-Platin(II)-Komplexe, in denen die Aminosäurereste die linksdrehenden Formen natürlich vorkommender α-Aminosäuren sind.

4. Arzneimittel, enthaltend neben üblichen Träger- und Verdünnungsmitteln einen cis-Platin(II)-Komplex gemäß Anspruch 1 als Wirkstoff.

5. Arzneimittel gemäß Anspruch 4, enthaltend den Wirkstoff in Form einer Lösung oder Suspension in Wasser oder einem polaren organischen Lösungsmittel.

6. Verfahren zur Herstellung von cis-Platin(II)-Komplexen gemäß Anspruch 1, dadurch gekennzeichnet, daß man
   a) einen Alkalichloro-Platin(II)-Komplex mit einem Aminozucker zum Molverhältnis 1 : 2 umsetzt

   oder daß man

   b) einen Alkaliiodo-Platin(II)-Komplex mit einem Aminozucker umsetzt und den so erhaltenen Iodo-Komplex der allgemeinen Formel $I_2PtLL'$ mit Silbernitrat und in Gegenwart von Wasser anschließend mit einem Alkalichlorid oder mit Silbernitrat oder Silbersulfat in einem organischen Lösungsmittel behandelt

   oder daß man

   c) einen Dihalogeno-Platin(II)-Bisaminosäure-Komplex in Gegenwart eines in der Peptidchemie üblichen Kupplungsreagenz mit einem Zucker mit freier OH-Gruppe oder einem Aminozucker mit freier $NH_2$-Gruppe umsetzt

   oder daß man

   d) einen Aminosäurezuckerester oder ein Aminosäurezuckeramid in der bei a) oder b) angegebenen Weise in den Platinkomplex überführt.

7. Verfahren nach Anspruch 6, dadurch gekennzeichnet, daß man im Zuckermolekül vorhandene reduzierend wirkende OH-Gruppen vor der Komplexbildung durch Veresterung, Veretherung oder Ketalisierung blockiert.

0059911

Europäisches Patentamt

# EUROPÄISCHER RECHERCHENBERICHT

Nummer der Anmeldung

EP 82 10 1532

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | JOURNAL OF THE CHEMICAL SOCIETY, CHEMICAL COMMUNICATIONS, Nr. 14, 21. Juli 1976, Seiten 598,599, J. DEHAND et al.: "Interaction of cis-diaminotolueneplatinum(II) with nucleosides: Evidence for guanosine O(6).N(7) chelation by platinum" * Seite 598 *<br>--- | 1,4 | C 07 H 23/00<br>A 61 K 31/70 //<br>C 07 F 15/00 |
| Y | FR-A-2 283 692 (ENGELHARD MINERALS & CHEMICALS CORP.) * Seiten 1-5 * & BE - A - 2 539 179<br>--- | 1,4 | |
| Y | CHEMICAL ABSTRACTS, Band 93, Nr. 17,27. Oktober 1980, Seite 711, Nr. 168561c, Columbus, Ohio, USA & JP - A - 80 47696 (TOYO JOZO CO., LTD.) 04-04-1980 * Zusammenfassung *<br>--- | 1,4 | RECHERCHIERTE SACHGEBIETE (Int. Cl. 3) |
| Y | DE-A-2 626 559 (BASF) * Seiten 1,2 *<br>--- | 1,4 | C 07 H 23/00<br>A 61 K 31/00<br>C 07 F 15/00 |
| Y | FR-A-2 459 247 (ENGELHARD MINERALS & CHEMICALS CORP.) * Seiten 24-25 * & DE - A - 3 022 957<br>--- | 1,4 | |
| Y | FR-A-2 423 479 (JOHNSON, MATTHEY & CO.) * Seiten 6,7 * & DE - A - 2 916 144<br>--- | 1,4 | |

Der vorliegende Recherchenbericht wurde für alle Patentansprüche erstellt.

| Recherchenort | Abschlußdatum der Recherche | Prüfer |
|---|---|---|
| DEN HAAG | 14-06-1982 | VERHULST W. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

0059911

Nummer der Anmeldung

EP 82 10 1532

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 001 126 (KIDANI, YOSHINORI) * Seiten 1-7 * --- | 1,4 | |
| Y | JOURNAL OF CLINICAL HEMATOLOGY AND ONCOLOGY, Band 7, Nr. 1, 1977, Seiten 114-134, M.L. TOBE et al.: "Structure, activity, reactivity and solubility relationships of platinum diamine complexes" * Seiten 114-124 * ----- | 4 | |

RECHERCHIERTE SACHGEBIETE (Int. Cl. 3)

Der vorliegende Recherchenbericht wurde für alle Patentansprüche erstellt.

| Recherchenort | Abschlußdatum der Recherche | Prüfer |
|---|---|---|
| DEN HAAG | 14-06-1982 | VERHULST W. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument